(19) Europäisches Patentamt
European Patent Office
Office européen des brevets

(11) EP 0 944 591 B1

(12) EUROPEAN PATENT SPECIFICATION

(45) Date of publication and mention of the grant of the patent:
**05.06.2002 Bulletin 2002/23**

(21) Application number: **97912320.5**

(22) Date of filing: **12.11.1997**

(51) Int Cl.[7]: **C07C 401/00**, G01N 33/82

(86) International application number:
**PCT/GB97/03097**

(87) International publication number:
**WO 98/21580 (22.05.1998 Gazette 1998/20)**

(54) **VITAMIN D IMMUNOASSAY SYSTEMS**

IMMUNTESTSYSTEM ZUR BESTIMMUNG VON VITAMIN D

SYSTEMES DE DOSAGES IMMUNOLOGIQUES DE LA VITAMINE D

(84) Designated Contracting States:
**AT BE CH DE DK ES FI FR GB IE IT LI LU NL PT SE**

(30) Priority: **13.11.1996 GB 9623569**

(43) Date of publication of application:
**29.09.1999 Bulletin 1999/39**

(73) Proprietor: **Immunodiagnostic Systems Limited**
**Tyne & Wear NE35 9PD (GB)**

(72) Inventors:
- **LAURIE, David, IDS Limited**
  **Boldon, Tyne & Wear NE35 9PD (GB)**
- **BARNES, Alexander, Kirkley, IDS Limited**
  **Boldon, Tyne & Wear NE35 9PD (GB)**

(74) Representative: **Allen, Philippa Margaret et al**
**Novagraaf Patents Limited**
**The Crescent**
**54 Blossom Street**
**York YO24 1AP (GB)**

(56) References cited:
**EP-A- 0 092 004**
**WO-A-80/02562**
**WO-A-94/26707**
**US-A- 4 585 741**

- **CHEMICAL ABSTRACTS, vol. 121, no. 9, 29 August 1994 Columbus, Ohio, US; abstract no. 109401, M. TANABE ET AL: "Preparation of Vitamin D Derivatives Containing a Substituent at the 25-Hydroxy Group" page 1143; column 1; XP002054205 & DATABASE WPI Section Ch, Week 9411 Derwent Publications Ltd., London, GB; Class B01, AN 94-089316 M. TANABE ET AL: & JP 06 041 058 (ASAHI CHEM IND CO LTD) , 15 February 1994 & AMERICAN CHEMICAL SOCIETY: "CHEMICAL ABSTRACTS FORMULA INDEX, C25H52O-Z, VOLUME 121 (PART 3 OF 3 PARTS)" 1994 , CHEMICAL ABSTRACTS , COLUMBUS OHIO US**
- **B. HOLLIS ET AL: "Determination of Vitamin D Status by Radioimmunoassay with an 125-Iodine Labelled Tracer" CLINICAL CHEMISTRY., vol. 39, no. 3, 1993, WINSTON US, pages 529-533, XP002054204**
- **CHEMICAL ABSTRACTS, vol. 123, no. 15, 9 October 1995 Columbus, Ohio, US; abstract no. 199203, H. WATANABE ET AL: "Synthetic studies of Vitamin D Analogs. 20. Synthesis of Tritiated 1.alpha.,25-Dihydroxy-22-oxavitamin D2" page 1295; column 1; XP002065444 & J. LABELLED COMPD. RADIOPHARM., vol. 36, no. 7, 1995, pages 645-654,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- CHEMICAL ABSTRACTS, vol. 107, no. 25, 21 December 1987 Columbus, Ohio, US; abstract no. 236332, R. SICINSKI ET AL: "Synthesis of 25-Hydroxy-[26,27-3H]-vitamin D2, 1.alpha.,25-Dihydroxy-[26,27-3H]-vitamin D2 and their (24R)-epimers" page 751; column 2; XP002065445 & ANALYTICAL BIOCHEMISTRY., vol. 161, no. 1, 1987, ORLANDO US, pages 96-102,
- CHEMICAL ABSTRACTS, vol. 97, no. 9, 30 August 1982 Columbus, Ohio, US; abstract no. 66509, J. CHANDLER ET AL: "Biosynthesis, Purification and Receptor Binding Properties of High Specific Radioactivity 1.alpha.,24(R),25-Trihydroxy-[26,27-methyl -3H]-vitamin D3" page 76; column 2; XP002065446 & JOURNAL OF STEROID BIOCHEMISTRY, vol. 16, no. 2, 1982, pages 303-310,
- CHEMICAL ABSTRACTS, vol. 91, no. 25, 17 December 1979 Columbus, Ohio, US; abstract no. 211666, K. BANNAI ET AL: "Synthesis of (24S)- and (24R)-1.alpha.-Hydroxy-cholecalciferol" page 711; column 2; XP002065447 & JOURNAL OF STEROID BIOCHEMISTRY, vol. 10, no. 4, 1979, pages 411-418,
- A. CALDAS ET AL: "The Simultaneous Measurement of Vitamin D Metabolites in Plasma: Studies in Healthy Adults and in Patients with Calcium Nephrolithiasis" JOURNAL OF LABORATORY AND CLINICAL MEDICINE , vol. 91, no. 5, 1978, pages 840-849, XP002065441
- CHEMICAL ABSTRACTS, vol. 89, no. 5, 31 July 1978 Columbus, Ohio, US; abstract no. 38985, S. YAMADA ET AL: "Synthesis of 25-Hydroxy[23,24-3H]-vitamin D3" page 259; column 2; XP002065448 & ANALYTICAL BIOCHEMISTRY., vol. 85, no. 1, 1978, pages 34-41,
- B. HALLORAN ET AL: "Isotopic Labeling Affects 1,25-Dihydroxyvitamin D Metabolism" BIOCHEMISTRY., vol. 28, no. 3, 7 February 1989, EASTON, PA US, pages 1278-1281, XP002065442
- J. NAPOLI ET AL: "Direct Chemical Synthesis of 1.alpha.,25-Dihydroxy[26,27-3H]vitamin D3 with High Specific Activity: Its Use in Receptor Studies" BIOCHEMISTRY., vol. 19, no. 11, 27 May 1980, EASTON, PA US, pages 2515-2521, XP002065443

## Description

The present invention relates to novel vitamin D derivatives, methods for the preparation of said derivatives, their application as tracers, and also to assay methods and kits which use said tracers.

More particularly, the present invention relates to novel vitamin D analogues which are modified at the 20-position, and to substituted derivatives thereof enabling quantitative detection thereof as a tracer.

### Background

Vitamin D is a commonly-used, collective term for a family of closely related molecules derived from naturally occurring 7-dehydrocholesterol (pro-vitamin D). Pro-vitamin D, primarily of dietary origin, undergoes photolytic conversion in the skin to parent vitamin $D_3$, (cholecalciferol) upon exposure to sunlight. This compound is biologically inactive, but enters the circulation and is hydroxylated in the liver to active 25-hydroxy vitamin D. A small proportion of this becomes further hydroxylated in the kidney to the highly potent calciotropic hormone 1,25-dihydroxy vitamin D.

1,25-Dihydroxy vitamin D is one of the major regulators of calcium (and phosphate) metabolism, stimulating intestinal calcium absorption and increasing bone resorption. It also inhibits parathyroid hormone (PTH) production both by direct action on the parathyroid glands and indirectly by raising serum calcium levels. 1,25-Dihydroxy vitamin D production is in itself stimulated by PTH, thus providing an effective control loop.

Hypovitaminosis D is commonly associated with dietary insufficiency of vitamin D, most frequently with vegetarianism. It is also associated with limited exposure to sunlight, (particularly the elderly and institutionalised) and skin pigmentation. In late-stage renal failure, 1α-hydroxylation may be impaired resulting in low 1,25-dihydroxy vitamin D levels. In hereditary vitamin D resistant rickets, there is end-organ resistance to 1,25-dihydroxy vitamin D and as a result 1,25-dihydroxy vitamin D levels are raised.

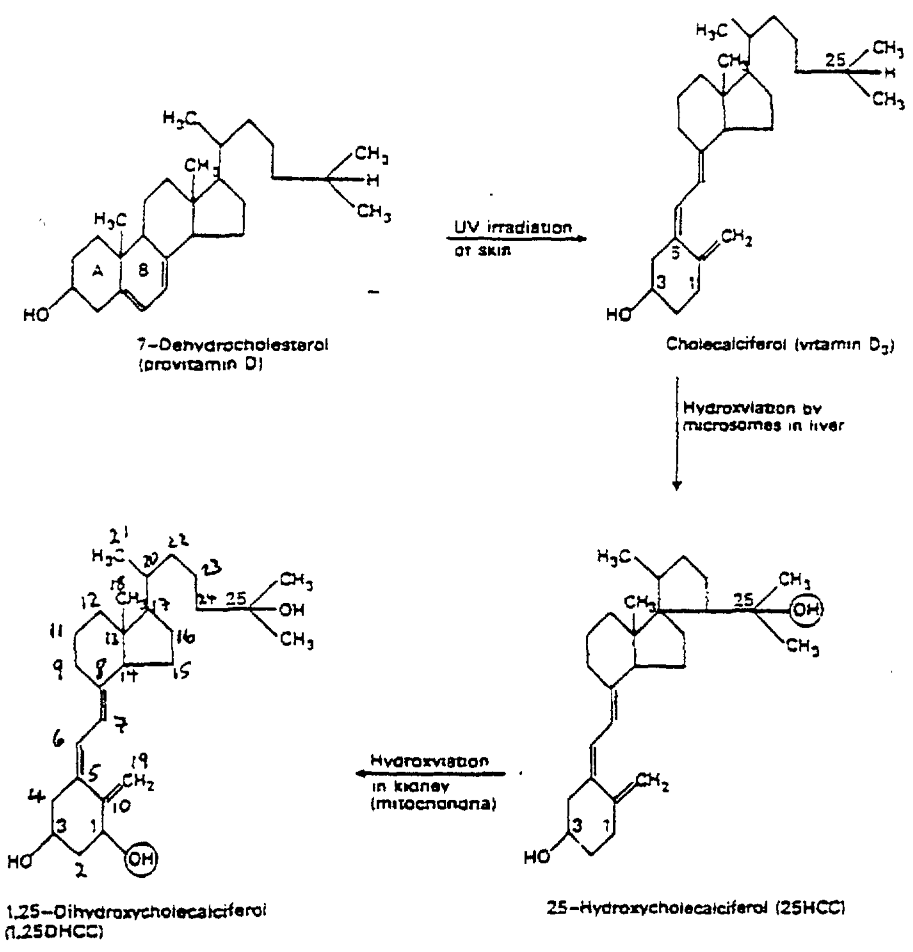


7-Dehydrocholesterol (provitamin D)

Cholecalciferol (vitamin $D_3$)

25-Hydroxycholecalciferol (25HCC)

1,25-Dihydroxycholecalciferol (1,25DHCC)

Rickets is a disease of children and is characterised by soft bones caused by insufficient calcium deposition. Accordingly, this causes the bones to bend under the weight of the body, leading to deformities. Rickets usually sets-in about the six months of life, and its intensity is directly related to the rapidity of bodily growth. There is conclusive evidence that the disease is due essentially to lack of vitamin D.

A disease related to rickets is Osteomalacia. Osteomalacia occurs in adults, particularly oriental women after they have experienced multiple pregnancies. The disease leads to bones becoming soft and deformed, and in similar fashion to rickets the serum calcium and inorganic phosphate levels are lowered and alkaline phosphatase is raised. In both diseases, administration of normal amounts of calcium and phosphate in the diet without administering vitamin D are ineffective. However, recovery from osteomalacia is rapid if vitamin D is administered in conjunction with calcium and phosphorous.

Accurate measurement of the hormonally active metabolite of vitamin D, 1,25-dihydroxy vitamin D is of clinical importance in monitoring renal and parathyroid disease, and is useful in understanding other disturbances of calcium metabolism, such as malignant hypercalcaemia and vitamin D intoxication.

Furthermore, the existence of the vitamin D receptor has been demonstrated in a number of locations other than the usual target tissues, such as the bone, intestine, and kidney. In particular receptors have been identified in the skin, breast, colon, brain, endocrine glands, muscle and haematopoietic cells. In addition, a large number of cancer cells of all organs possess vitamin D receptors, 1,25-dihydroxy vitamin D determining the transition from a state of proliferation to a state of differentiation. It thus behaves as an immuno-modulatory substance and regulates the growth and differentiation of normal cells and cancer cells. This may explain the epidemiological observations that children with rickets suffer immune deficiency, or the higher incidence of cancer of the colon associated with calcium and vitamin D deficiency, or prolonged survival of patients with breast cancer in which the vitamin D receptor is expressed.

A further disease which has been linked to vitamin D deficiency is osteoporosis. From the age of 40 years there is normally progressive loss of skeletal mass, which ultimately leads to senile osteoporosis. In women, osteoporosis occurs particularly after the menopause. There is no simple method of preventing senile osteoporosis, but physical effort and a diet rich in calcium and vitamin D may slow the rate of degeneration.

Prior Art

It will therefore be appreciated that determining circulating levels of 1,25-dihydroxy vitamin D in bodily fluids is of significant interest in the medical field and particularly in research and development. The assay of 1,25-dihydroxy vitamin D in human blood serves as an excellent indicator of the effectiveness of vitamin D metabolism in the body.

Development of assay methods for determining levels of 1,25-dihydroxy vitamin D have been slow, mainly due to the extremely low concentrations of 1,25-dihydroxy vitamin D in blood fluids. To date, the principle assay method for 1,25-dihydroxy vitamin D in serum is a competitive protein binding assay. One of the first assay methods to be developed utilised arduously-obtained and unstable rachitic chick intestinal binding protein[1]. Moreover, due to the relative lack of specificity of this cytosolic receptor, each sample required extensive chromatography before it could be used. Accordingly this restricted the assay procedure to only a few clinical laboratories.

Turning now to tracers for use in assays, which forms the inventive essence of this application. Typically, assay methods for 1,25-dihydroxy vitamin D necessitate the preparation of labelled derivatives of 1,25-dihydroxy vitamin D. Generally, such derivatives are labelled with a radioactive isotope or an enzyme. In addition, they need to possess high affinity and high specificity for the antibody which binds 1,25-dihydroxy vitamin D. A number of such tracers have been reported.

Tracers described in patent applications GB 1,589,921 and GB 1,592,170 claim tritiated 1,25-dihydroxy vitamin D for use in conjunction with polyclonal antibodies derived from the 3- or 25- substituted hemisuccinate esters of 1,25-dihydroxy vitamin D.

WO 80/02562 discloses C or H radiolabelled 1,25-dihydroxy vitamin D metabolic levels in blood and tissues, e.g. using competitive protein binding assay. A number of labelled analogues are disclosed.

US 5, 116, 573 discloses Vitamin D modified at the 6 and 19 positions by deuterium or tritium, and at the 25 position by hydroxy methyl or trifluorocarbon, which is resilient to metabolic cleavage, allowing tracing in the body and comparison of efficacy of modified vitamin.

Biochem 1980, 19, 2515-2521, Joseph, Napoli et al, discloses the synthesis of 26,27 tritium labelled 1,25 dihydroxy vitamin D, investigated using competitive protein binding assay.

Chemical Abstracts Vol. 57, no. 9, 30 August 1982, 66509g; Vol. 91, no. 25,17 December 1979 disclose in abstract a number of derivatives of Vitamin D, specifically, 1, 25-dihydroxy Vitamin D and isomers thereof, modified at the C20 position by tritiated alkyl substituents. The radio label seems to be used for mechanistic studies and activity determination.

US 4 585 741 discloses Vitamin D analogues which can be used in assays for the detection of Vitamin D3.

In addition, two of the most widely used radioimmunoassay kits for determining the concentration of 1,25-dihydroxy vitamin D, utilized tritiated 1,25-dihydroxy vitamin D as tracer. These kits manufactured by Nichols and Incstar use tritiated 1,25-dihydroxy vitamin D, in conjunction with the thymus receptor protein as binder.

However, the use of tritiated tracers in radioimmunoassay kits suffers from a series of drawbacks. The major drawback in using tritiated tracers in radioimmunoassay methodology is the lengthy liquid scintillation counting (LSC) stage necessary to determine the concentration of the bound tracer. Typically for 60 assay tubes this process takes ten hours to complete.

Furthermore, the preparation of samples prior to determining the concentration of tritium by LSC is time consuming, involving complex mixing procedures.

Yet a further problem with tracers based on tritiated derivatives is that they require Recovery Determination as an essential step in the assay method, to ensure accurate results. Typically, this results in a further time delay of 45 minutes preparation time, and 3.3 hours involved in the LSC stage.

The Nichols assay uses the following procedure for Recovery Determination. In the Nichols assay, each sample is spiked with a small amount of tritiated 1,25-dihydroxy vitamin D and samples extracted by a $C_{18}$/solvent method. Part of each sample extract is counted to measure extracted tritiated 1,25-dihydroxy vitamin D and calculate the recovery on extraction, typically 50-70%. The remaining sample extract goes into a tritium radioreceptor assay using calf thymus receptor and charcoal separation followed by liquid scintillation counting. Patient sample values are then obtained by correcting each assay result for the extraction recovery.

It will be appreciated from the above, that kits based on tritium tracers can only achieve a relatively low sample throughput due to the lengthy times involved in sample preparation and counting. Typically only 12 to 24 samples can be analysed by one person, with the results being obtained by the end of the third day. In addition, methods based on tritiated derivatives require manual result calculation, which needs to be corrected for Recovery Determination.

The tracers proposed in the patent application US 5,232,836 are based on novel 11-substituted iodine 125 derivatives of 1,25-dihydroxy vitamin D. This document also describes the use of these 11-substituted iodine tracers in conjunction with an antibody which is also derived from 11-substituted derivatives of 1,25-dihydroxy vitamin D. This patent document goes on to describe comparative assay trials in which the iodinated tracer was found to give superior results compared with assays using tritiated tracers. This superiority was particularly noticeable when applied to measuring very low concentrations of 1,25-dihydroxy vitamin D. Furthermore, the comparative results showed a much lower background variation when using the iodinated tracer. It will therefore be appreciated that iodinated tracers when applied to vitamin D assays possess superior characteristics of performance than tritiated tracers.

However a drawback of the 11-substituted iodine 1,25-dihydroxy vitamin D derivatives described in US 5,232,836 is that antibodies that are compatible with the 11-substituted derivatives do distinguish between the endogenous form of vitamin D, vitamin D3 and semi-synthetic form, vitamin D2. As vitamin D2 is used in pharmaceutical preparations and vitamin D2 is metabolised by the same mechanism as the endogenous form, producing 1,25-dihydroxyvitamin D2 as the active metabolite, a useful assay for 1,25-dihydroxyvitamin D should be able to measure the contribution made by the D2 form. Antibodies raised against 11-position vitamin D3 analogues do not cross-react with the D2 form and therefore cannot provide a true picture of a patient's vitamin D status.

GB 1, 592,170 discloses analogues linked to the immunogen protein via the 25-position, and investigates whether they give similar cross reactivity with 1,25 dihydroxyvitamin D2 and D3. Mawer et al (Clinica. Chimica Acta, 1990, 1902, 199-210) entitled "A sensitive radioimmunoassay using a monoclonal antibody that it equipotent for ercalcitriol and calcitriol" discloses that antibodies do exist which can cross react with D2 and D3.

It is a further drawback of analogues linked to the immunogen protein via the 25-position that their synthesis is not adapted for the convenient study or production thereof. For example the 11-substituted derivative described in US 5,232,836 can only be produced via a long and convoluted chemical synthesis. The chemical synthesis described in this document involves manufacturing the vitamin D derivative in two halves, and then coupling of the two halves using the Wittig reaction to generate the double bond at the 7,8 position of vitamin D.

Accordingly, this lengthy and complex synthesis prevents wide-spread use of 11-substituted iodinated derivatives in radio-immuno assay methodology.

WO 94/26707 relates to Vitamin D amide derivatives, of a first general formula having seven sub classes covering variation in the cyclo hexylidene ring, and four sub-classes representing variation in Group Y at position 22 to 24. This huge diversity is moreover exemplified by 24 variants specifically, and by innumerable variants generically by combination of the different groups.

WO 94/26707 discloses their cell modulating activity, use as antagonists of Vitamin D and in vivo metabolites. The compounds are envisaged for use in chemotherapy, treatment of bone disease and a wealth of possible medical applications, at page 11 bridging page 12.

Although iodine is used as part of the synthetic route, no mention is made of substitution by $^{125}$I or equivalent for use in immunoassay.

Chemical Abstracts Vol. 121, no. 9, 29 August 1994, 109401z discloses a 1, 25-dihydroxy substituted Vitamin D derivative comprising $^{125}$I label. The derivative, registered as registry number 156515-30-3 comprises diether linking

groups between the Vitamin D and a benzocarbamide substituent, and methylene groups between the benzo and carbamide groups. It is not clear if the compound is used for assay, as disclosed for the unmodified $^{125}I$ analogue.

Problems with the Prior Art

It is a problem with the prior art that tritiated tracers emit low energy -radiation which is difficult to detect, requires long detection periods (of up to 10 hours for 60 assay tubes) for accurate measurement and decays relatively rapidly.

It is a further problem with the prior art that laborious and time consuming LSC methods are required to measure a concentration of bound tritiated tracer.

It is also a problem with the prior art that kits based on tritiated derivatives require Recovery Determination as an essential step, and that this results in a time delay of 45 minutes for preparation time and 3.3 hours counting time.

It is also a problem with the prior art that kits based on tritiated derivatives require manual result calculation, which needs to be corrected for Recovery Determination.

It is a problem with the prior art that kits based on tritiated derivatives are practically limited to low throughputs, the results taking long periods to obtain. Typically, results will be obtained on the end of the third day.

It is also a problem with the prior art that there is no iodine derivative of an analogue of vitamin D which can bind to an antibody that is existing or is readily produced and can detect both the D2 and D3 forms of 1,25-dihydroxy vitamin D.

We have now surprisingly found that the above problems can be solved in admirable manner according to the present invention.

In its broadest aspect there is provided in accordance with the present invention an analogue of 1,25-dihydroxy vitamin D, modified at the $C^{20}$ position, which can be produced readily and will bind to at least an existing or readily produced antibody, wherein the analogue comprises a substituent adapted for quantitative detection. Preferably the analogue comprises a radioactive substituent containing iodine 125.

In a further aspect of the invention there is provided a process for the preparation of such analogue, and novel intermediates prepared by the process.

In a further aspect of the invention there is provided a tracer for use in vitamin D immunoassay methodology which tracer comprises an analogue as hereinbefore defined.

In a further aspect of the invention there is provided a composition comprising a compound of general formula I as hereinbefore defined as a tracer, optionally in admixture with a diluent, carrier, solvent, adjuvant, stabiliser preservative, or dispersant.

In a further aspect of the invention there is provided a method for determining the concentration of 1,25-dihydroxy vitamin D with use of an analogue as hereinbefore defined. Preferably such method comprises the immunoextraction of samples such as serum and plasma samples, preparation of assay mixture of a tracer as hereinbefore defined and samples, and the assay thereof by quantitative detection. Preferably the method employs radiation detection.

In a further aspect of the invention there is provided a radioimmunoassay kit comprising an analogue as hereinbefore defined. It is an advantage that the radioassay results obtained with use of the kit do not require Recovery Determination, and therefore enable a more rapid radioimmunoassay procedure, for example the results can be read directly from a radiation detector such as a gamma counter and involve negligible time to calculate.

It has been found that the radioimmunoassay kit of the invention can accommodate a high throughput of greater than 100 samples, and the results can be obtained within a shorter time than can typically be achieved with standard tritiated radioimmunoassay kits.

Preferably an analogue as hereinbefore defined comprises a compound of general formula I:

$$A\ {}^{20}CRR^1$$

wherein

A = 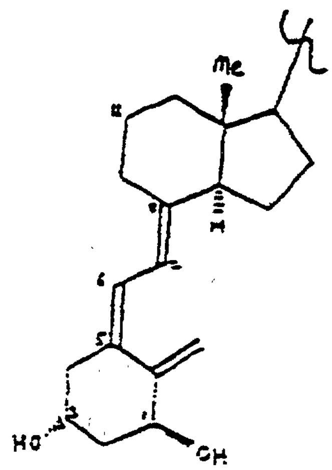

R is a radioactive substituent; and
$R^1$ is selected from H and alkyl.

Preferably

R represents a group - $CR^2R^3NR^4R^5$

wherein

$R^2$, $R^3$ and $R^4$ are selected from H, alkyl or one of $R^2$ and $R^3$ form an unsaturated bond with $R^4$; and
$R^5$ represents the group YX $(CH_2)_n$ Q

wherein

each of Y and X is independently selected from a single bond, - O -, - C(=O) -, - NH -, - $O(CH_2)_mC(=O)N$ -, - O $(CH_2)_mC(=O)$ - and - O $(CH_2)_mN$ -;
n is a whole number integer selected from 0 to 15, for example from 1 to 6; and
Q is an iodine 125- containing homo- or hetero-, mono- or poly-aromatic or alicyclic containing group, selected from optionally substituted benzene, naphthalene, imidazole such as histamine, pyrrole, pyrazole, pyridine, pyrimidine, (iso)quinoline, purine, oxazole, thiazole, and thiophene, wherein optional substituents include hydroxy, halo, alkyl such as methyl, amino, or cyano.

Preferably

$R^1$ = H or $CH_3$

Most preferably

R is - CH = N - or - $CH_2NH$ - ;
Y is selected from a single bond and - $OCH_2C(=O)$ - ;
X is selected from - C(=O) - and - NH - ;
n is 1 or 2 ; and
Q is hydroxy iodo benzyl or is iodo imidazolyl.

It is a further advantage that an analogue of the invention may be obtained by a short and high yielding process.
Preferably there is provided a process for preparing a compound of general formula as hereinbefore described which comprises the reaction of a compound of formula II:

$$AC^{20}HR^1CR^2R^3NR^4YL$$

with a compound of formula III:

$$L^1X(CH_2)_nQ$$

wherein L and $L^1$ are leaving groups.

In a particular advantage of the invention the reaction of compounds of formula II and III is a nucleophilic substitution reaction with elimination of $H^+$ and $OH^-$, for example one of - YL and $L^1X$ - is an amino containing group and one is a carbonyl containing group. The reaction is carried out according to techniques and under conditions as known in the art.

Compounds of formulae II and III may be obtained by any means known in the art. Preferably there is provided a process for the preparation of a compound of formula II which comprises the reaction of a compound of formula IV:

$$AC^{20}HR^1CHO$$

with a compound of formula V:

$$HNR^4Y$$

under appropriate conditions as known in the art.

The compound of formula III may be obtained with use of known reaction techniques.

The compound of formula IV may be obtained by any means known in the art.

Preferably there is provided a process for the preparation of a compound of formula IV which comprises the oxidation of a compound of formula VI, 1 - hydroxycyclovitamin $D_2$:

(VI)

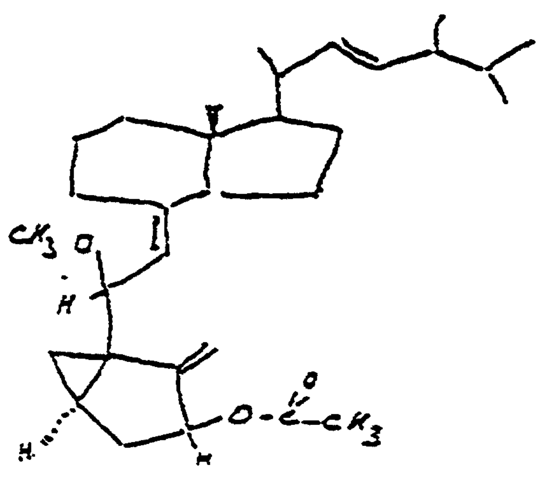

with ozonised oxygen, triphenyl phosphine and basic solution in sequence.

According to a further aspect of the invention there is provided any intermediate compound of formula II, III, IV and VI as hereinbefore defined.

In a further aspect of the invention there is provided the use of an intermediate compound as hereinbefore defined to produce a compound of general formula I as hereinbefore described.

Reference herein to any compound of the invention is to the compound having structure as indicated, or to a precursor, metabolite or other derivative or functional equivalent thereof.

A composition of the invention as hereinbefore defined may be in any suitable form adapted for use in an immunoassay method as hereinbefore defined, and preferably in a form adapted for inclusion in an immunoassay kit as hereinbefore defined.

The radioimmunoassay kit as hereinbefore defined may include any suitable additional components and preferably includes an inhibitor adapted to isolate substantially all of the material to be analysed in a sample to be subjected to immunoassay. Preferably the sample to be assayed is plasma or is serum and the inhibitor comprises a component as known in the isolation of thyroxine assays, for example as disclosed in US Patents 3,911,096 and 3,928,553

The immunoextraction may be carried out in known manner, by immobilisation, or liquid phase extraction and subsequent precipitation by suitable means.

It is a particular advantage that the use of an inhibitor, and in particular of a preferred inhibitor as defined renders the immunoassay technique of the invention with improved reliability and accuracy.

The kit suitably comprises duplicating components enabling the duplication for verification and accuracy purposes of each sample tested. It is a particular advantage that the time - efficient method of the invention enables duplication in convenient manner.

The invention is now illustrated in non limiting manner with reference to the following examples and corresponding schemes:

Example 1 - Preparation of Intermediates II & IV (Scheme 1)

1 -Hydroxycyclovitamin $D_2$-1-acetate (compound 1) was prepared from Vitamin $D_2$ (egrocalciferol) by the method of Paarens, *et al* (Proc. Natl. Acad. Sci. USA, 1978, 75, 2080-1).

*Compound 2 (Intermediate IV)*

Compound 1 (5.0g, 11mmol) in dichloromethane/methanol (10:1, 100mL) was cooled to <-60°C and treated with ozonised oxygen until starting material was consumed as indicated by TLC. The reaction vessel was purged with nitrogen gas, triphenyl phosphine added (4.3g) and the mixture allowed to warm to room temperature. The solution was washed with saturated sodium hydrogen carbonate solution and the solvent evaporated. The residue was redissolved in 1,4-dioxan (20mL) and an aqueous solution of toluene sulphonic acid (0.2mL, 25mg/mL) added and the mixture heated to 50°C for 15 minutes. The reaction was quenched with saturated $NaHCO_3$ solution and extracted with 3 portions of diethyl ether.

The organic phase was washed with saturated $NaHCO_3$ solution, saturated NaCl solution, dried over $MgSO_4$ and the solvent removed in vacuo. The residue was loaded on a silica column (50g) equilibrated with 2% methanol in dicloromethane. The solvent composition was changed to 5% methanol and the first 50mL was collected and solvent evaporated to give compound 2.

*Compound 3 (Intermediate II)*

Compound 2 (1.8g) was treated with methyl carboxymethoxylamine (1.0g) in methanol (50mL) containing 5% triethylamine for 24 hours at room temperature. The solvent was evaporated and residue chromatographed on silica (ethyl acetate / ether 7:3) to yield compound 3. NMR, ppm, 0.52 (3H,s, 18-H3), 1.17 (3H,d,J=7Hz, 21-H3), 2.03 (3H, s, 1-OCOCH3), 3.80 (3H,s, -CO2CH3), 4.30 (1H,m, 3 -H), 4.49 (1H,m, 1- H), 4.58 (2H,s, -OCH2CO2CH3), 5.02 (1H, m (sharp), 19(Z)-H), 5.36 (1H,m (sharp), 19(E)-H), 6.02 (1H,d,J=11Hz, 7-H), 6.38 (1H,d,J=11Hz, 6-H), 7.40 (1H,d, J=7Hz, 22-H).

*Compound 4 (Intermediate II)*

Compound 3 (0.65g, 1.4mmol) was dissolved in methanol under an atmosphere of nitrogen, 1M sodium hydroxide (2.0mL) added and the mixture was stirred at room temperature for 3 hours. The solvent was evaporated in vacuo and the residue treated with 0.5M sodium phosphate buffer, pH 6.0 and extracted with 3 portions of diethyl ether. The organic phase was washed with saturated NaCl solution, dried over anhydrous $Na_2SO_4$. The solvent was evaporated and the residue chromatographed on silica (ethyl acetate / methanol, 10:1) to give compound 4 (0.38g, 0.9mmol). NMR, ppm, 0.51 (3H,s, 18-H3), 1.18 (3H,d,J=7Hz, 21-H3), 4.26 (1H,m, 3 -H), 4.48 (1H,m, 1 -H), 4.63 (2H,s, -OCH2CO2H), 5.03 (1H,m (sharp), 19(Z)-H), 5.38 (1H,m (sharp), 19(E)-H), 6.05 (1H,d,J=10Hz, 7-H), 6.40 (1H,d, J=10Hz, 6-H), 7.43 (1H,d, J=7Hz, 22-H). M/z 416 ($M-H^+$, FAB in glycerol).

Example 2 - Preparation of Compound of formula (I) (Scheme 2)

A solution of 90µg of Compound 4 in 90µL of 1,4 dioxane was allowed to react with 6.0µL of a solution of 1 part tri-butylamine and 20 parts 1,4 dioxane at 8-10°C for 5 minutes. 6.0µL of a solution of 1 part iso-butyl chloroformate and 40 parts 1,4 dioxane was added to the reaction mixture and incubated at 6-12°C for 30 minutes. This was mixture 1, containing predominantly the (iso-butyl)formate anhydride of compound 4.

A solution of 3µg of histamine dihydrochloride in 5µL 0.5 M Sodium phosphate buffer pH 7.5, was reacted with 50µg of chloramine T in 5.0µL of water in the presence of 2.5mCi Iodine-125 (sodium iodide in dilute solution, pH 7-11, at 100mCi/mL on reference date), for 5 minutes at 18-24°C. A solution of 250µg of sodium metabisulphite in 5µL of water was added, followed by 2.5µL of a 0.1M solution of sodium hydroxide. This was mixture 2, containing histamine and $^{125}$I-Iodohistamine.

Mixture 1 (68µL) was added to the entire volume of Mixture 2 and allowed to react for 90 minutes at 0-5°C. 1mL of a saturated sodium bicarbonate solution was added and the product extracted with three 1mL portions of ethyl acetate. The organic phase was washed twice with 1.5mL of saturated sodium bicarbonate. Hexane (0.5mL) was added and the solution washed with 1.5mL of water. The organic phase was added to a glass test tube and the solvent evaporated at 37°C under a gentle stream of nitrogen gas. The residue was re-dissolved in methanol, diluted to 50% methanol with water and applied to a Hypersil-ODS (C18, 4.6 x 150 mm, 3µ particle size) high pressure liquid chromatography column. The product was eluted in a gradient of 50:50 methanol : water containing 0.04% trifluoroacetic acid to 100% methanol. The fractions corresponding to the radioactive peak containing were pooled to give 370 Ci of compound 5.

Compound 5 was determined to have a specific activity of 1.8 x $10^6$ Ci/mole by the method of Chiang (Clinical Chemistry, 1987, 33, 1245).

Example 3 - Preparation of Intermediate II (Scheme 3)

*Compound 6 (Intermediate II)*

Compound 2 (0.5g) was dissolved in methanol under an atmosphere of nitrogen. Ammonium chloride (0.2g) was added followed by 0.2mL of a solution of sodium Cyanoborohydride in 5M sodium hydroxide. The mixture was stirred at room temperature for 2 hours, then 1.0mL of 5M sodium hydroxide added and stirring continued for a further 3 hours. The solvent evaporated in vacuo and the residue treated with 20mL of 0.5M sodium phosphate buffer pH 8.0 and the mixture extracted with 3 portions of diethly ether. The organic phase was washed with saturated NaCl solution, and dried over anhydrous $Na_2SO_4$. The solvent was evaporated and the residue chromatographed on silica (methanol/chloroform/triethylamine 50:10:1) to give compound 6 (0.12g).

Scheme 4 illustrates the preparation of a Compound 7 of the formula (I).

Example 4 - Method for Determining the Concentration of Vitamin D in Serum and Plasma Samples

*Sample Preparation* - Serum and plasma samples were delipidated by treatment with a dextran sulphate/magnesium chloride reagent and centrifuged to give a delipidated sample. The delipidated supernatant must be absolutely clear. Grossly lipaemic samples or samples that have been subjected to more than 4 freeze-thaw cycles may not give a clear supernatant. Samples that do not give a clear delipidated supernatant are not suitable for immunoextraction. Difficult samples can often be clarified by centrifugation at a higher g-force or for a longer time.

A vitamin D binding protein inhibitor comprising a solution of 8-anilino-1-naphthalene sulphonic acid was present to ensure that all analyte, i.e. vitamin D, present in the sample was available.

*Immunoextraction* - The immunoextraction device comprises a capsule containing a suspension of solid phase to which is attached a monoclonal antibody highly specific for 1,25-Dihydroxy vitamin D. The delipidated sample was added to the capsule, rotated end-over-end for 3 hours and then the column was washed. 1,25-Dihydroxy vitamin D was eluted directly into a glass assay tube by three portions of elution reagent. The elution reagent was then evaporated under a flow of nitrogen gas. As the volume of solvent used is small, the time required for evaporation is only 15-20 minutes. The residue was dissolved in assay buffer.
These tubes were used directly in the assay.

*Iodine 125 Radioimmunoassay* - Primary antibody was added to tubes containing calibrators and to the tubes containing the extracts. The tubes were incubated overnight at 2-8C. The following day, tracer comprising Compound 5 was added, incubated at room temperature for a further 2 hours and the Sac Cel solid phase second antibody added. After 30 minutes, 4mL of wash solution was added to dilute the assay mixture and tubes were centrigued. The liquid was decanted, tubes allowed to drain and the iodine 125 activity remaining in the pellets counted. A calibration curve gave serum 1,25-dihydroxy vitamin D values for unknowns directly.

Compound 5 was demonstrated to bind to antibodies such as AS7 described by Wildermuth, et al (Clinica Chimica Acta 1993, 220, 61) and monoclonal antibodies such as 5F2 described by Mawer et al (Steriods, 1985, 46, 741). In each case binding was inhibited by 1,25-dihydroxyvitamin D3 and by 1,25-dihydroxyvitamin D2.

The method of this example was carried out in convenient and simple manner and was found to give highly accurate and reproducible results.

Further advantages of the invention are apparent from the foregoing.

## Claims

1. Compound of the formula I

$$(I) \qquad A\ ^{20}CRR^1$$

Wherein A is

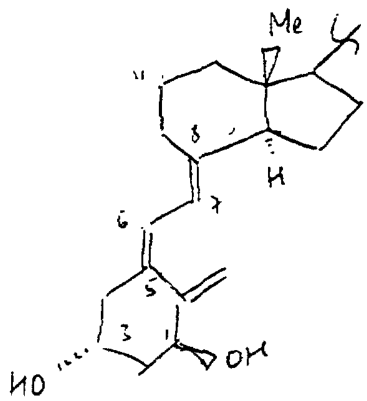

$R^1$ is hydrogen or alkyl and
R represents a group

$$CR^2R^3NR^4R^5$$

wherein

$R^2$, $R^3$ and $R^4$ are selected from H, alkyl or one of $R^2$ and $R^3$ form an unsaturated bond with $R^4$; and
$R^5$ represents the group YX $(CH_2)_n$ Q

wherein each of Y and X is independently selected from a single bond, - O -, - C(=O) -, - NH -, - $O(CH_2)_mC(=O)N$-, - $O(CH_2)_mC(=O)$ - and - O $(CH_2)_mN$ -;
n is a whole number integer selected from 0 to 15; and Q is an iodine 125-containing homo- or hetero-, mono- or poly-aromatic or alicyclic containing group, selected from optionally substituted benzene, naphthalene, imidazole, histamine, pyrrole, pyrazole, pyridine, pyrimidine, (iso)quinoline, purine, oxazole, thiazole and thiophene, wherein the optional substituents include hydroxy; halo, alkyl (such as methyl), amino and cyano.

2. Compound as claimed in Claim 1 wherein $R^1$ is selected from H and $CH_3$.

3. Compound as claimed in any of Claims 1 and 2 wherein:

R is - CH = N - or - $CH_2NH$ - ;
Y is selected from a single bond and - $OCH_2C(=O)$ - ;
X is selected from - C(=O) - and - NH - ;
n is 1 or 2 ; and
Q is hydroxy iodo benzyl or is iodo imidazolyl.

4. Process for preparing a compound of general formula I as claimed in any one of Claims 1 to 3 which comprises the reaction of a compound of formula II:

$$AC^{20}HR^1CR^2R^3NR^4YL$$

with a compound of formula III:

$$L^1X(CH_2)_nQ$$

wherein L and $L^1$ are leaving groups.

5. Process as claimed in Claim 4 which is a nucleophilic substitution reaction with elimination of $H^+$ and $OH^-$, wherein II, or one of YL and $L^1X$ - is an amino containing group and $R^5$ or one of YL and $L^1X$ is a carbonyl containing group.

6. Process for the preparation of a compound of formula II which comprises the reaction of a compound of formula IV:

$$AC_{20}HR^1CHO$$

with a compound of formula V:

$$HNR^4YH$$

under appropriate conditions as known in the art.

7. Process for the preparation of a compound of formula IV which comprises the oxidation of a compound of formula VI, 1 alpha - hydroxycyclovitamin $D_2$:

(VI)

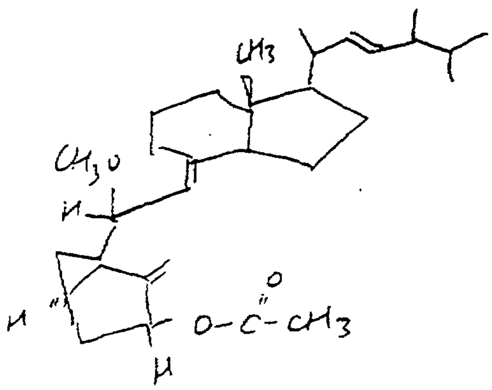

with ozonised oxygen, triphenyl phosphine and basic solution in sequence.

8. A novel intermediate compound of formula II, III, IV and VI as hereinbefore defined in any one of Claims 4 to 7.

9. Use of an intermediate compound as in any of Claims 4 to 7 to produce a compound of general formula I as hereinbefore described in any of Claims 1 to 3.

10. A tracer for use in vitamin D immunoassay methodology which tracer comprises a compound as hereinbefore defined in any one of Claims 1 to 3.

11. A composition comprising a compound of formula I as hereinbefore defined in any one of Claims 1 to 3, or a tracer as hereinbefore defined in Claim 10 in admixture with a diluent, carrier, solvent, adjuvant, stabiliser, preservative or dispersant.

12. A tracer composition comprising as tracer a compound of formula

$$A^{20}CRR^1$$

as defined in any of Claims 1 to 3, for use in vitamin D immunoassay methodology.

**13.** Composition as claimed in any of Claims 11 - 12 adapted for use in an immunoassay method comprising an immunoassay kit.

**14.** Composition as claimed in any of Claims 11 to 13 which additionally includes an inhibitor.

**15.** Method for determining the concentration of 1,25-dihydroxy vitamin D with use of compound, composition or inhibitor as hereinbefore defined in any one of Claims 1 to 3 or 11 to 14 which comprises the immunoextraction of samples such as serum and plasma samples, preparation of assay mixture of a tracer as hereinbefore defined and samples comprising a compound or composition as defined in any of Claims 1 to 3 or Claims 11 to 14, and the assay thereof by quantitative detection.

## Patentansprüche

**1.** Verbindung der Formel I

$$(I) \qquad A^{20}CRR^1,$$

worin A

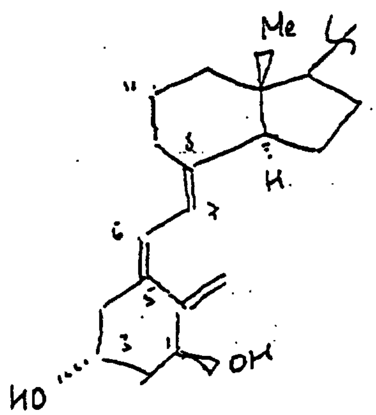

und $R^1$ Wasserstoff oder Alkyl ist
und R eine Gruppe

$$CR^2R^3NR^4R^5$$

darstellt, worin

$R^2$, $R^3$ und $R^4$ unter H und Alkyl ausgewählt sind oder entweder $R^2$ oder $R^3$ eine ungesättigte Bindung mit $R^4$ bildet;
und $R^5$ die Gruppe

$$YX(CH_2)_nQ$$

darstellt, worin

Y und X beide unabhängig voneinander unter Einfachbindung, -O-, -C(=O)-, -NH-, $-O(CH_2)_mC(=O)N-$, $-O(CH_2)_mC(=O)-$ und $-O(CH_2)_mN-$ ausgewählt sind;
n eine zwischen 0 und 15 gewählte ganze Zahl;

und
Q eine Iod-125 enthaltende, homo- oder hetero-, mono- oder polyaromatische oder alicyclische Gruppen enthaltende Gruppe, ausgewählt unter wahlweise substituiertem Benzol, Naphthalin, Imidazol, Histamin, Pyrrol, Pyrazol, Pyridin, Pyrimidin, (Iso)chinolin, Purin, Oxazol, Thiazol und Thiophen, worin the wahlweise vorhandenen Substituenten Hydroxy, Halogen, Alkyl (wie zum Beispiel Methyl), Amino und Cyano einschliessen.

**2.** Verbindung wie in Anspruch 1 beansprucht, worin $R^1$ unter H und $CH_3$ ausgewählt ist.

**3.** Verbindung wie in einem der Ansprüche 1 und 2 beansprucht, worin
R -CH=N- oder $-CH_2NH-$ ist;
Y unter Einfachbindung und $-OCH_2C(=O)-$ ausgewählt ist;
X unter -C(=O)- und -NH- ausgewählt ist,
n 1 oder 2 ist; und
Q Hydroxyiodbenzyl oder Iodimidazolyl ist.

**4.** Verfahren zur Darstellung einer Verbindung der allgemeinen Formel I, wie in einem beliebigen der Ansprüche 1 bis 3 beansprucht, das die Reaktion einer Verbindung der Formel II:

$$AC^{20}HR^1CR^2R^3NR^4YL$$

mit einer Verbindung der Formel III:

$$L^1X(CH_2)_nQ$$

umfasst, worin L und $L^1$ austretende Gruppen sind.

**5.** Verfahren wie in Anspruch 4 beansprucht, das eine nukleophile Substitutionsreaktion unter Eliminierung von $H^+$ und $OH^-$ ist, worin II oder entweder YL oder $L^1X$- eine Amino enhaltende Gruppe und $R^5$ oder entweder YL oder $L^1X$ eine Carbonyl enthaltende Gruppe ist.

**6.** Verfahren zur Darstellung einer Verbindung der Formel II, das die Reaktion einer Verbindung der Formel IV:

$$AC_{20}HR^1CHO$$

mit einer Verbindung der Formel V:

$$HNR^4YH$$

unter geeigneten Bedingungen, wie sie fachbekannt sind, umfasst.

**7.** Verfahren zur Darstellung einer Verbindung der Formel IV, das die Oxidation einer Verbindung der Formel VI, 1-Alpha-Hydroxycyclovitamin $D_2$:

(VI)

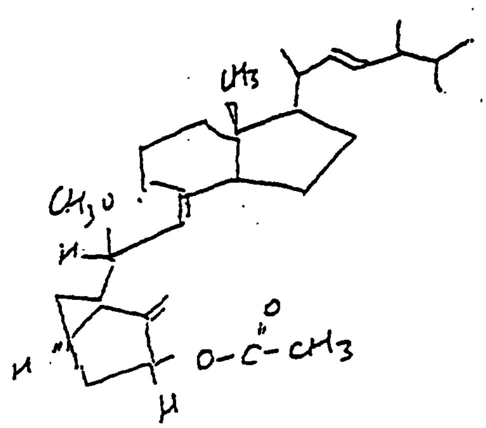

aufeinanderfolgend mit ozonisiertem Sauerstoff, Triphenylphosphin und basischer Lösung umfasst.

**8.** Neuartige Zwischenverbindung der Formel II, III, IV und VI, wie vorstehend in einem beliebigen der Ansprüche 4 bis 7 definiert.

**9.** Verwendung einer Zwischenverbindung wie in einem beliebigen der Ansprüche 4 bis 7 zur Herstellung einer Verbindung der allgemeinen Formel I, wie vorstehend in einem beliebigen der Ansprüche 1 bis 3 beschrieben.

**10.** Tracer zur Verwendung in der Methodologie des Immunoassays von Vitamin D, wobei dieser Tracer eine wie vorstehend in einem beliebigen der Ansprüche 1 bis 3 definierte Verbindung umfasst.

**11.** Zusammensetzung, eine Verbindung der Formel I, wie sie vorstehend in einem beliebigen der Ansprüche 1 bis 3 definiert ist, oder einen Tracer, wie er vorstehend in Anspruch 10 definiert wurde, in Mischung mit einem Verdünnungsmittel, Träger, Lösemittel, Adjuvans, Stabilisator, Konservierungsmittel oder Dispergiermittel umfassend.

**12.** Tracerzusammensetzung zur Verwendung in der Methodologie des Immunoassays von Vitamin D, als Tracer eine Verbindung der Formel

$$A\ {}^{20}CRR^{1},$$

wie sie in einem beliebigen der Ansprüche 1 bis 3 definiert ist, umfassend.

**13.** Zusammensetzung, wie in einem beliebigen der Ansprüche 11 bis 12 beansprucht, zur Verwendung in einer Immunoassaymethode mit einer Immunoassay-Ausrüstung.

**14.** Zusammensetzung, wie in einem beliebigen der Ansprüche 11 bis 13 beansprucht, die zusätzlich einen Inhibitor einschliesst.

**15.** Verfahren zur Bestimmung der Konzentration von 1,25-Dihydroxy-vitamin D unter Verwendung einer Verbindung, Zusammensetzung oder eines Inhibitors, wie vorstehend in einem beliebigen der Ansprüche 1 bis 3 oder 11 bis 14 definiert, das die Immunextraktion von Proben wie Serum oder Plasma, die Zubereitung der Assaymischung aus einem Tracer, wie er vorstehend definiert ist, und Mustern, eine Verbindung oder Zusammensetzung umfassend, wie sie in einem beliebigen der Ansprüche 1 bis 3 oder Ansprüche 11 bis 14 definiert ist, und dessen Assay durch quantitativen Nachweis umfasst.

## Revendications

**1.** Composé de formule I

(I) $A\ {}^{20}CRR^1$

Dans laquelle A est

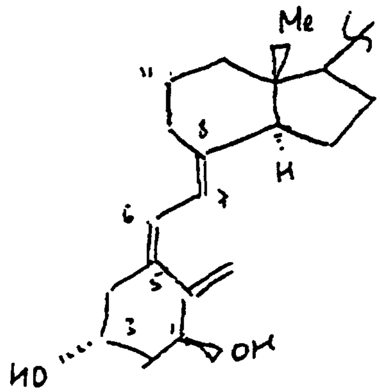

$R^1$ est un atome d'hydrogène ou un groupe alkyle et
R représente un groupe

$$CR^2R^3NR^4R^5$$

où

$R^2$, $R^3$ et $R^4$ sont choisis parmi H, un groupe alkyle ou un de $R^2$ et $R^3$ forme une liaison insaturée avec $R^4$ ; et $R^5$ représente le groupe $YX(CH_2)_nQ$
où chacun de Y et X est, indépendamment l'un de l'autre, choisi parmi une simple liaison, -O-, -C(=O) -, -NH-, $-O(CH_2)_mC(=O)N-$, $-O(CH_2)_mC(=O)$ - et $-O(CH_2)_mN-$ ;
n est un nombre entier choisi parmi 0 à 15 ; et Q est un groupe homo- ou hétéro-, mono- ou poly-aromatique ou alicyclique, contenant de l'iode 125, choisi parmi le benzène, le naphtalène, l'imidazole, l'histamine, le pyrrole, le pyrazole, la pyridine, la pyrimidine, l'(iso)quinoléine, la purine, l'oxazole, le thiazole et le thiophène éventuellement substitués, où les substituants éventuels incluent des groupes hydroxy, halogéno, alkyle (tel que méthyle), amino et cyano.

**2.** Composé selon la revendication 1, où $R^1$ est choisi parmi H et $CH_3$.

**3.** Composé selon l'une quelconque des revendications 1 et 2, où :

R est -CH=N- ou $-CH_2NH-$ ;
Y est choisi parmi une simple liaison et $-OCH_2C$ (=O) - :
X est choisi parmi -C(=O)- et -NH- ;
n vaut 1 ou 2 ; et
Q est un groupe hydroxyiodobenzyle ou un groupe iodoimidazolyle.

**4.** Procédé pour préparer un composé de formule générale I selon l'une quelconque des revendications 1 à 3 qui comprend la réaction d'un composé de formule II :

$$AC^{20}HR^1CR^2R^3NR^4YL$$

avec un composé de formule III :

$$L^1X(CH_2)_nQ$$

dans lesquelles L et $L^1$ sont des groupes quittants.

**5.** Procédé selon la revendication 4, qui est une réaction de substitution nucléophile avec élimination de $H^+$ et $OH^-$, où II, ou un de YL et $L^1X$- est un groupe contenant une amine et $R^5$ ou un de YL et $L^1X$ est un groupe contenant un carboxyle.

**6.** Procédé pour la préparation d'un composé de formule II qui comprend la réaction d'un composé de formule IV :

$$AC_{20}HR^1CHO$$

avec un composé de formule V :

$$HNR^4YH$$

dans des conditions appropriées telles que connues dans la technique.

**7.** Procédé pour la préparation d'un composé de formule IV, qui comprend l'oxydation d'un composé de formule VI, la 1-alpha-hydroxycyclovitamine $D_2$ :

(VI)

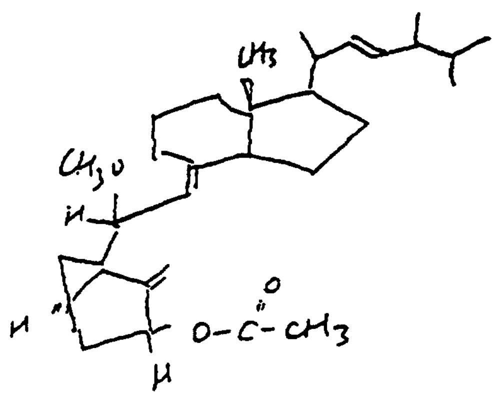

avec, en séquence, de l'oxygène ozonisé, une triphénylphosphine et une solution basique.

**8.** Nouveau composé intermédiaire de formules II, III, IV et VI selon l'une quelconque des revendications 4 à 7 ci-dessus.

**9.** Utilisation d'un composé intermédiaire selon l'une quelconque des revendications 4 à 7 pour produire un composé de formule générale I, selon l'une quelconque des revendications 1 à 3 ci-dessus.

**10.** Traceur pour une utilisation dans une méthodologie de dosage immunologique de la vitamine D, traceur qui comprend un composé selon l'une quelconque des revendications 1 à 3 ci-dessus.

**11.** Composition comprenant un composé de formule I selon l'une quelconque des revendications 1 à 3 ci-dessus, ou traceur selon la revendication 10 ci-dessus, en mélange avec un diluant, un véhicule, un solvant, un adjuvant, un stabilisant, un conservateur ou un dispersant.

**12.** Composition de traceur comprenant en tant que traceur, un composé de formule

$$A\,^{20}CRR^1$$

selon l'une quelconque des revendications 1 à 3, pour une utilisation dans une méthodologie de dosage immunologique de la vitamine D.

**13.** Composition selon l'une quelconque des revendications 11 et 12, adaptée pour une utilisation dans une méthode de dosage immunologique comprenant une trousse de dosage immunologique.

**14.** Composition selon l'une quelconque des revendications 11 à 13, qui inclut en plus un inhibiteur.

**15.** Procédé pour déterminer la concentration en 1,25-dihydroxy-vitamine D en utilisant un composé, une composition ou un inhibiteur selon l'une quelconque des revendications 1 à 3 ou 11 à 14 ci-dessus, qui comprend l'immunoextraction d'échantillons tels que des échantillons de sérum et de plasma, la préparation d'un mélange de dosage d'un traceur tel que défini ci-dessus et d'échantillons comprenant un composé ou une composition selon l'une quelconque des revendications 1 à 3 ou des revendications 11 à 14, et son dosage par détection quantitative.

fig.1

SCHEME 1

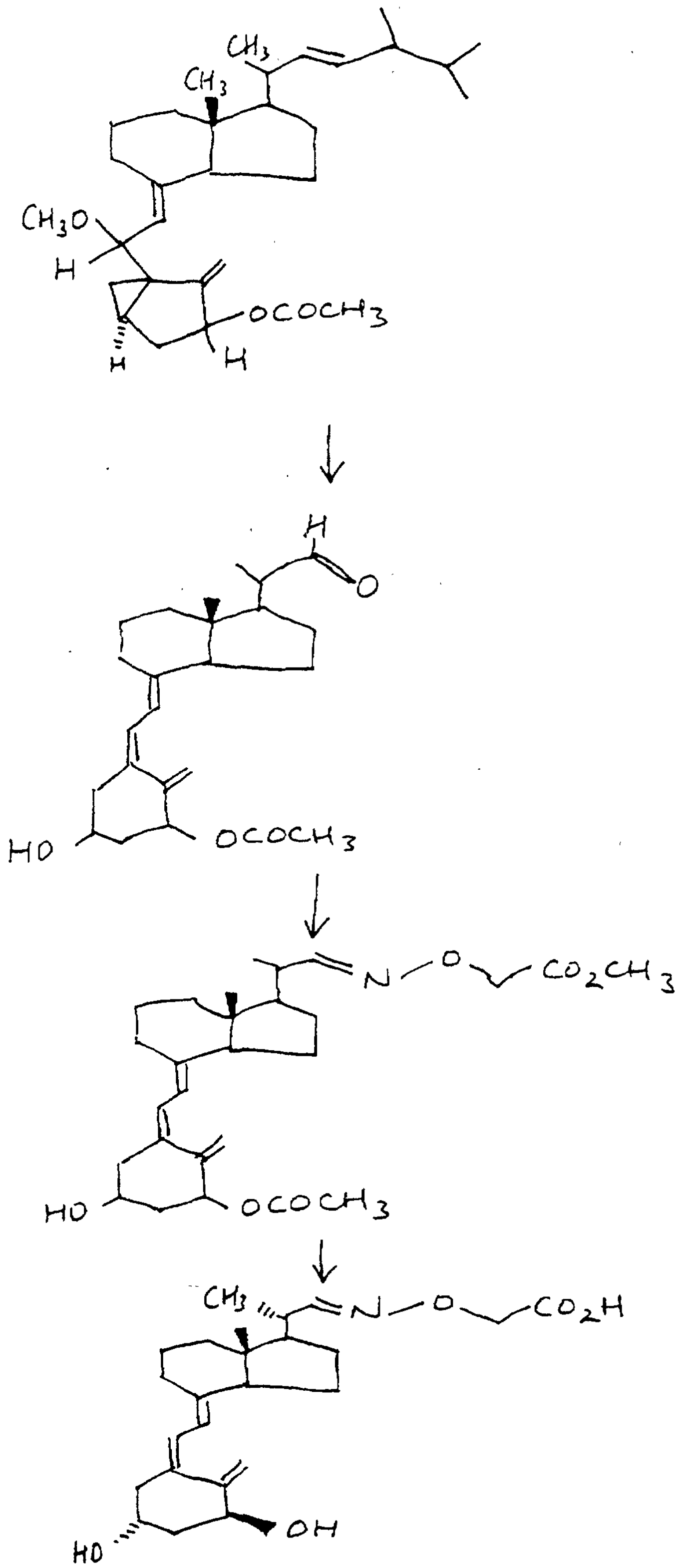

Compound 1

Compound 2

Compound 3

Compound 4

Fig 2

SCHEME 2

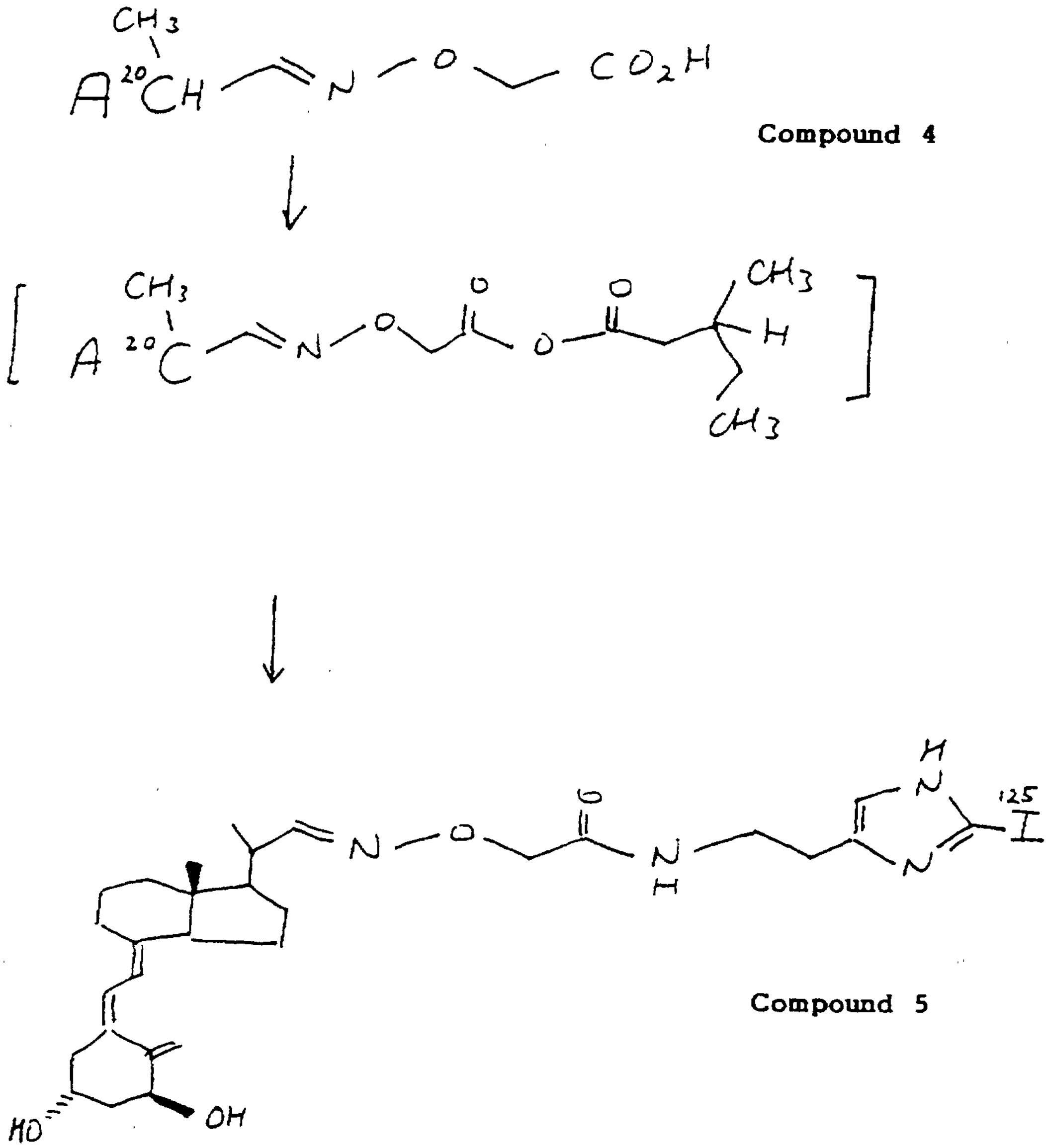

Compound 4

Compound 5

fig. 3

SCHEME 3

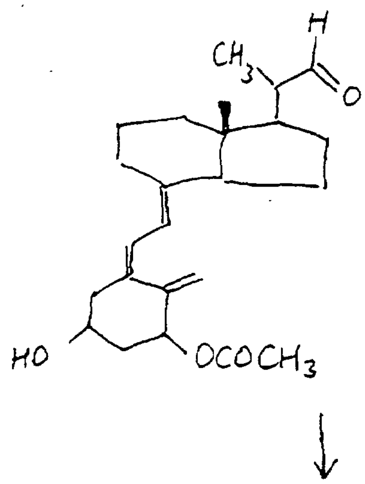

Compound 2

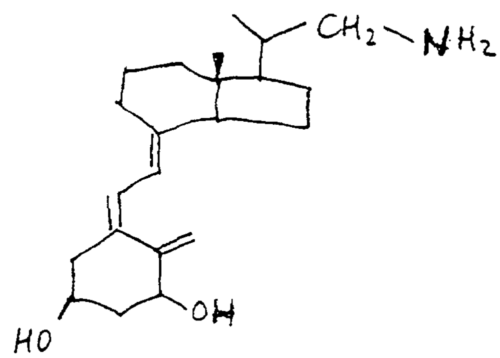

Compound 6

SCHEME 4

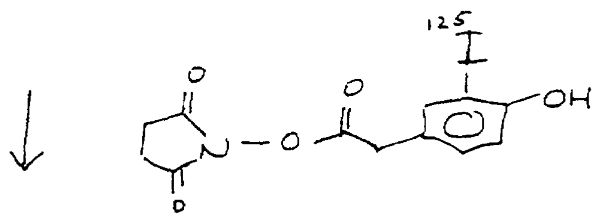

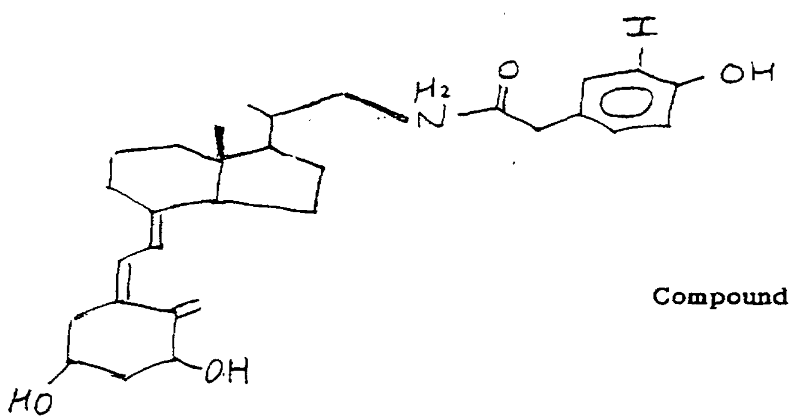

Compound 7